# EUROPEAN PATENT SPECIFICATION

(11) **EP 2 614 812 B1**
(45) Date of publication and mention of the grant of the patent: **08.06.2016**
(21) Application number: 13150734.5
(22) Date of filing: 10.01.2013
(51) Int. Cl.: A61K 8/23, A61K 8/43, A61K 8/67, A61K 8/73, A61Q 11/00

(54) **Mouthwash**
Mundwasser
Bain de bouche

(30) Priority: 10.01.2012 IT MI20120019
(43) Date of publication of application: 17.07.2013
(73) Proprietor: Castellaccio, Restituta, 21047 Saronno (VA) (IT)
(72) Inventor: Castellaccio, Restituta, 21047 Saronno (VA) (IT)
(74) Representative: Montelatici, Linda Anna

(56) References cited:
- EP-A1- 0 444 492
- EP-A1- 1 340 490

## Description

The present invention relates to a mouthwash for oral hygiene based on chlorhexidine and in particular a mouthwash that prevents the formation of plaque on the teeth by avoiding the known drawback of their dark pigmentation.

The solutions for oral hygiene based on chlorhexidine have long been known, as this compound is a potent antibacterial due to its ability to penetrate the outer membrane of bacteria and coagulate their internal proteins. Chlorhexidine also carries out an intense anti-plaque activity. The solutions containing chlorhexidine at various concentrations are also used to prevent postoperative complications.

However, the prolonged use of mouthwashes based on chlorhexidine in oral hygiene causes the formation of an undesirable brownish pigmentation on teeth.

It is also known from European patent EP 1340490 in the name of the Applicant a mouthwash comprising chlorhexidine, ascorbic acid and sodium metabisulfite, having the advantageous property of a reduced occurrence of the unwanted pigmentation.

The object of the present invention is to provide a new mouthwash for oral hygiene comprising chlorhexidine, having an improved anti-pigmentation activity compared to that of known mouthwashes. This object is achieved with the mouthwash having the characteristics specified in claim 1. Further details of such mouthwash are specified in the subsequent claims.

The mouthwash according to the present invention comprises chlorhexidine and is therefore effective against gingivitis, bacterial plaque and periodontitis, but does not cause unwanted side effects. It has been surprisingly discovered, in fact, that the association of hyaluronic acid, or a salt or a complex thereof, of ascorbic acid and a metabisulfite salt is more effective in neutralizing the pigmentation activity of chlorhexidine, than only metabisulfite salt.

Moreover, thanks to the presence of hyaluronic acid or a salt thereof, the mouthwash according to the present invention also performs a protective action of the oral mucosa and healing of possible wounds of the oral cavity.

Further advantages and characteristics of the mouthwash according to the present invention will become clear to those skilled in the art from the following description of some embodiments thereof.

The mouthwash according to the present invention comprises chlorhexidine, or a salt or a complex thereof, a metabisulfite salt of alkali metal or alkaline earth metal, ascorbic acid and hyaluronic acid or a salt or a complex thereof.

The mouthwash also comprises in known manner a suitable solvent medium, preferably water.

As salt of chlorhexidine, in the mouthwash according to the present invention can be used for example chlorhexidine digluconate or chlorhexidine diacetate. Preferably, the mouthwash according to the present invention comprises chlorhexidine digluconate.

According to a preferred embodiment of the invention the chlorhexidine, or said salt or complex thereof, is contained in mouthwash in an amount comprised between 0.05% and 0.30% by weight on the total volume of the mouthwash. Preferably, the amount by weight of chlorhexidine, or of said salt or complex thereof, is between 0.1% and 0.2%.

As metabisulfite salt of alkali metal or alkaline earth metal, the mouthwash according to the present invention may comprise for example sodium, potassium, or calcium metabisulfite salt.

Preferably, the mouthwash according to the present invention comprises sodium metabisulfite.

The amount by weight of metabisulfite salt of alkali metal or alkaline earth metal is preferably comprised between 0.1% and 0.5% on the total volume of the mouthwash. Preferably, the amount by weight of metabisulfite salt of alkali metal or alkaline earth metal is comprised between 0.15% and 0.3% by weight on the total volume of the mouthwash.

In a preferred aspect, the mouthwash according to the present invention further comprises ascorbic acid and sodium citrate tribasic.

Preferably, the amount of ascorbic acid in the mouthwash according to an aspect of the present invention is comprised between 0.1% and 1% by weight, while the amount of sodium citrate tribasic is preferably comprised between 0.8% and 2.0% by weight with respect to the total volume of mouthwash.

As a salt or a complex of hyaluronic acid, the mouthwash according to the present invention can comprise for example sodium hyaluronate, potassium hyaluronate and preferably a mixture of hyaluronates with low and high molecular weight. The amount by weight to volume of hyaluronic acid, or salt or complex thereof, is preferably comprised between 0.05% and 1.2% by weight to volume of solution, more preferably between 0.1% and 0.2%.

The mouthwash according to the present invention can further comprise one or more additional components selected from the group consisting of fluorides of alkali metals or alkaline earth metals. Preferably, the mouthwash comprises from 0.05 to 0.1% of sodium fluoride.

The mouthwash according to the present invention may further comprise one or more additives, selected from the group consisting of sweeteners, flavorings, wetting agents, preservatives, emulsifiers, pH regulators, food colouring.

As sweeteners, the mouthwash according to the present invention can comprise for example xylitol, sodium saccharin, acesulfame potassium, sucralose, stevia extract.

As flavorings, the mouthwash according to the present invention can comprise for example peppermint, menthol, anethole, viridis mint, cinnamon, cloves, eucalyptol.

As humectants, the mouthwash according to the present invention can comprise for example propylene glycol, sorbitol, glycerin.

As preservatives, the mouthwash according to the present invention can comprise for example sodium benzoate, methylisothiazolinone.

As surfactants solubilizing, the mouthwash according to the present invention can comprise for example: hydrogenated castor oil Peg 40, Poloxamer 407.

As pH regulators, the mouthwash according to the present invention can comprise for example sodium citrate, citric acid.

As colouring, the mouthwash according to the present invention can comprise for example CI 19140, CIU 42090, CI 17200.

According to a preferred embodiment, the mouthwash according to the invention comprises the following ingredients:
- 1.: Water
- 2.: Xylitol
- 3.: Propylene Glycol
- 4.: Hydrogenated Castor Oil PEG 40
- 5.: Ascorbic acid
- 6.: Chlorhexidine digluconate
- 7.: Sodium hyaluronate
- 8.: Aroma
- 9.: Poloxamer 407
- 10.: Sodium metabisulfite
- 11.: Sodium citrate
- 12.: Citric acid
- 13.: C.I. 42090
- 14.: C.I. 17200

## Claims

1. Mouthwash comprising chlorhexidine or a salt or complex thereof, a alkaline or alkaline earth metal metabisulfite salt, ascorbic acid, **characterized by** comprising hyaluronic acid or a salt or complex thereof.

2. Mouthwash according to claim 1, **characterized in that** said hyaluronic acid or salt or complex thereof is in the form of a mixture of sodium and/or potassium hyaluronate of low and high molecular weight.

3. Mouthwash according to claim 1 or 2, **characterized by** comprising one or more further components selected in the group consisting of alkaline and alkaline earth metal fluorides.

4. Mouthwash according to claim 2, **characterized by** comprising 0.05% to 0.30% by weight of chlorhexidine, 0.1% to 1% by weight of ascorbic acid, 0.1% to 0.5% of sodium metabisulfite and 0.05% to 1.2% by weight of hyaluronic acid or a salt or complex thereof.

5. Mouthwash according to claim 2 or 3, **characterized by** comprising 0.05% to 0.1% of sodium fluoride.

6. Mouthwash according to claim 1 or 2, **characterized by** comprising one or more additives selected in the group consisting of sweeteners, flavors, moistening agents, preservatives, emulsifiers, pH regulators, food colouring agents.

## Patentansprüche

1. Mundwasser, umfassend Chlorhexidin oder ein Salz oder einen Komplex davon, ein Alkali- oder Erdalkalimetall-Metabisulfit-Salz, Ascorbinsäure, **dadurch gekennzeichnet, dass** es ferner Hyaluronsäue oder ein Salz oder einen Komplex davon umfasst.

2. Mundwasser nach Anspruch 1, **dadurch gekennzeichnet, dass** die besagte Hyaluronsäure oder ein Salz oder Komplex davon die Form einer Mischung von Natrium- und/oder Kalium-Hyaluronat mit niedrigem und hohem Molekulargewicht hat.

3. Mundwasser nach Anspruch 1 oder 2, **dadurch gekennzeichnet, dass** es eine oder mehrere, aus der Gruppe bestehend aus Alkali- oder Erdalkalimetall-Fluoriden ausgewählte Komponenten umfasst.

4. Mundwasser nach Anspruch 2, **dadurch gekennzeichnet, dass** es 0,05 bis 0,30 Gew.-% Chlorhexidin umfasst, 0,1 bis 1 Gew.-% Ascorbinsäure, 0,1 bis 0,5 Gew.-% Natrium-Metabisulfit und 0,05 bis 1,2 Gew.-% Hyaluronsäure oder ein Salz oder ein Komplex davon.

5. Mundwasser nach Anspruch 2 oder 3, **dadurch gekennzeichnet, dass** es 0,05 bis 0,1 Gew.-% Natriumfluorid umfasst.

6. Mundwasser nach Anspruch 1 oder 2, **dadurch gekennzeichnet, dass** es ein oder mehrere, aus der Gruppe bestehend aus Süßungsmitteln, Geschmacksstoffen, Feuchthaltemitteln, Konservierungsmitteln, Emulgatoren, pH-Regulatoren, Lebensmittelfarbstoffen, ausgewählte Additive umfasst.

## Revendications

1. Bain de bouche comprenant de la chlorhexidine ou un sel ou un complexe de celle-ci, un sel de métabisulfite de métaux alcalins ou alcalino-terreux, de l'acide ascorbique, **caractérisé en ce qu'**il comprend de l'acide hyaluronique ou un sel ou un complexe de celui-ci.

2. Bain de bouche selon la revendication 1, **caractérisé en ce que** ledit acide hyaluronique ou un sel ou un complexe de celui-ci est sous la forme d'un mélange d'hyaluronate de sodium et/ou potassium de poids moléculaires élevé et faible.

3. Bain de bouche selon la revendication 1 ou 2, **caractérisé en ce qu'**il comprend un ou plusieurs composants supplémentaires choisis dans le groupe constitué par les fluorures de métaux alcalins et alcalino-terreux.

4. Bain de bouche selon la revendication 2, **caractérisé en ce qu'**il comprend de 0,05 % à 0,30 % en poids de chlorhexidine, de 0,1 % à 1 % en poids d'acide ascorbique, de 0,1 % à 0,5 % de métabisulfite de sodium et de 0,05 % à 1,2 % en poids d'acide hyaluronique ou d'un sel ou d'un complexe de celui-ci.

5. Bain de bouche selon la revendication 2 ou 3, **caractérisé en ce qu'**il comprend de 0,05 % à 0,1 % de fluorure de sodium.

6. Bain de bouche selon la revendication 1 ou 2, **caractérisé en ce qu'**il comprend un ou plusieurs additifs choisis dans le groupe constitué par les édulcorants, les aromatisants, les agents hydratants, les conservateurs, les émulsifiants, les régulateurs de pH, les colorants alimentaires.
